# EUROPEAN PATENT APPLICATION

(11) **EP 4 851 625 A1**
(43) Date of publication of application: **09.12.2026**
(21) Application number: 26183183.8
(22) Date of filing: 04.06.2026
(51) Int. Cl.: G01N 33/14

(54) **COMPUTER-IMPLEMENTED METHOD FOR MONITORING WINE IN A BOTTLE**

(30) Priority: 05.06.2025 IT 202500013045
(71) Applicant: Nanotec S.r.l., 98076 Sant'Agata di Militello (ME) (IT)
(72) Inventor: GENTILE, Roberto, 98070 ACQUEDOLCI (ME) (IT)
(74) Representative: Locas, Davide

(57) **Abstract**

A computer-implemented method, and apparatus, for monitoring wine in a bottle during the respective cold chain, by means of a monitoring device applied directly to each bottle. The method uses an artificial intelligence model to evaluate the storage conditions of the wine, ensuring traceability and transparency.

## Description

The present invention relates to a computer-implemented method and an apparatus for monitoring bottled wine during a respective cold chain.

### STATE OF THE ART

For those working in the high-quality agri-food sector, and in particular in the fine wine sector, it is important to be able to objectively ensure that the correct environmental parameters of the respective cold chain are maintained throughout the supply chain, from production to delivery to the end consumer.

Every year, a significant number of fine wines are affected by damage caused by improper transport and storage, which has a direct impact on the quality of the product and its market value. For producers of premium wines, ensuring optimal climatic conditions throughout the cold chain is extremely important for preserving the wine's organoleptic characteristics and integrity.

Specifically, the cold chain in the wine sector consists of the following consecutive links, in which it is essential to ensure compliance with certain environmental standards, otherwise the quality of the product will be compromised:
1. Production and bottling department;
2. Storage in a temperature-controlled facility, logistics by the manufacturer;
3. Loading area for dispatch;
4. Transport requiring a specific contract to ensure compliance with the cold chain;
5. Unloading at the logistics centre for receipt and distribution;
6. Storage on shelves and counters in points of sale;
7. Purchase, transport and storage by the end consumer.

Once the wine has been produced and bottled, ensuring that the product remains in perfect condition, retaining its organoleptic characteristics intact as the wine matures naturally over time, depends on the ability to manage the aforesaid stages whilst adhering to the optimal parameters for temperature, humidity and bottle positioning.

It is therefore important for wine producers, for a number of reasons relating to the quality assurance of their product, to be able to objectively guarantee that the wine produced and released onto the market has not been subjected to thermal or humidity-related stress, or stress arising from its position inside the individual bottle.

In the current state of the art, there are several known methods for monitoring the environmental conditions to which a wine consignment is subjected; however, these methods entail a number of drawbacks. Firstly, monitoring is generally carried out using sensors that detect the environmental conditions to which a consignment of wine as a whole is exposed, without evaluating the actual conditions to which the individual bottles of wine are subjected.

When applied to the entire consignment, such monitoring is in fact unreliable and does not guarantee, that is to say, it does not allow for the control of the physical parameters of temperature, humidity and position on which the storage and integrity of the wine contained inside each individual bottle depend. In fact, the current system is limited to the step to which it is applied, excluding monitoring of the preceding step, from bottling to handover to the carrier, and also excluding the subsequent step, from delivery to the logistics or reception and distribution centre to the final destination; furthermore, it suffers from the inevitable approximation in data measurement, which consequently loses its objectivity, as such systems are unable to measure and guarantee the same parameters for individual bottles of wine; the data instead represent an average of the conditions prevailing throughout the area where several bottles are stored (for example, a container has an area of approximately 65 m²).

This is a significant issue given that a single sensor used to detect temperature and humidity inside a container is unable to verify the conditions of an individual bottle which, unlike the others, may have been placed in areas more or less exposed to different temperatures and humidity levels. Furthermore, even with this "collective" control system, producers can only monitor the first four links in the cold chain; it is then the responsibility of the retail sector to monitor links five and six (Unloading at the logistics reception and distribution centre; Storage on shelves and counters in points of sale), whilst the seventh and final link (Purchase, transport and storage by the end consumer) remains the responsibility of the end consumer, who is free to continue such monitoring. However, end customers often lack the means to ensure effective and comprehensive monitoring of the storage conditions of individual bottles of wine when stored, for example, in a cellar at home.

In a nutshell, it is possible to track an entire shipment - be it a pallet or a container - but only between the dispatch warehouse and the receiving warehouse for distribution. However, in this case, the customer has no way of verifying the actual climatic and environmental conditions to which the individual bottle of wine has been subjected.

Existing environmental monitoring systems are therefore generally applied to batches or entire containers, without any direct monitoring of individual bottles. This approach has significant limitations, as fluctuations in temperature, humidity and position affecting a single bottle can compromise its quality, without this being detected by the methods currently in use.

Furthermore, relationships between the conditions to which a particular bottle of wine has been subjected and any potential deterioration in its quality are highly complex, and depend in part on the type of wine itself. In this regard, although the monitoring systems currently available detect physical parameters related to wine storage, they are unable to reliably evaluate the actual final quality of the product. Consequently, they are also unable to guarantee that the quality of the wine has indeed been maintained during the respective cold chain.

An example of a known bottle monitoring system is described in patent application EP 3291155 A1. This system allows for the tracking of individual bottles, but it too has a number of drawbacks. Firstly, it is designed to monitor the bottle solely during the ageing process of the liquid inside it, and is not suitable for monitoring throughout the entire cold chain. Furthermore, it results in significant energy consumption by the monitoring devices used, which consequently limits the duration of monitoring or reduces the number of data points that can be recorded. Although it measures various physical parameters that influence the proper storage of the monitored bottle, this system merely evaluates each of them individually, comparing them with their respective pre-set nominal values, and is therefore not effective in capturing the complex relationships between multiple physical parameters and the actual degradation of the monitored product.

Given the current state of the art, there is therefore a clear need for a wine monitoring system that enables effective tracking of a bottle of wine throughout the entire cold chain and provides a sufficiently reliable guarantee that the quality of the wine has remained essentially unchanged.

### SUMMARY OF THE INVENTION

The technical problem underlying the present invention is thus to provide a computer-implemented method, and an apparatus, for monitoring wine in a bottle during a respective cold chain, which is functionally and structurally designed to at least partially overcome one or more of the drawbacks mentioned above with reference to the cited prior art.

In particular, one of the aims of the invention is to provide a method and apparatus that enable the effective monitoring of bottled wine throughout its entire cold chain.

Another aim of the invention is to provide a method and apparatus that enable the monitoring of storage conditions, even for individual bottles containing wine.

A further aim of the invention is to provide a method and apparatus that improve the management of bottled wine throughout the entire supply chain, minimizing, as far as possible, any deterioration in quality caused by sub-optimal storage conditions.

Another aim of the invention is to provide a method and apparatus that enable the environmental storage conditions of an individual bottle of wine to be monitored reliably and accurately.

Another aim of the invention is to provide a method and apparatus for the early detection and management of storage conditions that are detrimental to the quality of the wine.

A further aim of the invention is to provide a method and apparatus that enable continuous monitoring of bottled wine, even over very long periods of time.

An aim of the invention is to provide a method and an apparatus capable of reliably demonstrating the state of storage of a product, certifying its quality and guaranteeing a competitive advantage to manufacturers and retailers, and greater safety and awareness to consumers when making a purchase.

This problem is at least partially resolved, and one or more of the aforementioned aims are achieved, at least in part, by the invention, in a first aspect thereof, by means of a computer-implemented method for monitoring wine in a bottle during a respective cold chain. The method preferably involves training a first artificial intelligence model to generate a quality index representative of storage conditions of the wine in the bottle as a function of a plurality of predefined physical parameters. The predefined physical parameters should preferably relate to the wine in the bottle, and even more preferably comprise at least two of the following: temperature, humidity and the spatial orientation of the bottle itself. It is also preferable for the predefined physical parameters to include all three of the parameters listed above-temperature, humidity and spatial orientation-relating to the bottle containing the wine. In some embodiments, the predefined physical parameters may also comprise other quantities, as described in more detail below.

The quality index is preferably designed to identify conditions that could compromise one or more qualitative and/or quantitative parameters of the wine in the bottle.

It should be noted that, in the context of the present invention, the term "qualitative and/or quantitative parameters of the wine in the bottle" preferably refers to those measurable parameters which an expert in the field of oenology would recognize as being objectively and closely linked to the quality of the wine. These parameters may, for example, comprise the oxidation index, the concentration of polyphenols and phenolic compounds, the possible presence of harmful volatile compounds, such as sulphides or volatile phenolic compounds, the concentration of sulphur dioxide, the alcohol content, colour, total acidity, the aromatic profile resulting from the concentration of compounds such as esters, alcohols, benzenoids, aldehydes, norisoprenoids and terpenes, the pH and/or the antioxidant activity of the wine.

Preferably, the aforementioned method comprises supplying a monitoring device that includes a sensor group with sampling frequencies that are preferably adjustable.

It is preferable that the method comprises applying the monitoring device to the bottle containing wine.

Preferably, the method comprises detecting predefined physical parameters relating to the wine in the bottle using the sensor group, thereby obtaining a data set. The data set preferably comprises a time series for each of the predefined physical parameters detected.

Preferably, the method comprises providing the input data set to the first artificial intelligence model trained as described above.

Preferably, the method comprises generating the quality index based on the data set using the first artificial intelligence model trained as described above.

Preferably, the method comprises providing a user with that quality index or a processed version thereof.

It should be noted that, in the context of the present invention, the term "user" preferably refers to at least one of the following: the wine producer, the wine transporter, the wine retailer, the wine shop managing the wine, the wine purchaser, or any other operators, private individuals or managers involved in one or more links of the wine cold chain.

The method described above, thanks to the monitoring device applied to the wine bottle, makes it possible to directly detect the predefined physical parameters relating to each individual bottle. This makes it possible to monitor the actual conditions to which a specific bottle has been subjected with greater precision and reliability. Furthermore, the monitoring device can remain applied to the bottle for its entire lifespan, ensuring continuous monitoring throughout all the links in the wine's cold chain. In fact, the end consumer has the option of purchasing the sensor device applied to the bottle along with the bottle itself, so that they can continue to monitor and keep track of the storage conditions of the wine they have purchased.

The use of an artificial intelligence model also makes it possible to resolve, or at least mitigate, the problem arising from the complex correlations between the physical parameters of bottle storage and the qualitative and/or quantitative parameters that determine the quality of the wine. As there is no need for complex programming or prior configuration - during which it would be virtually impossible to account for all possible scenarios across all types of wine - training a dedicated artificial intelligence model allows for a highly reliable estimation of the quality index associated with wine storage conditions, effectively assessing any potential risks involved, and yielding reliable estimates even without having previously considered all possible combinations of scenarios. Furthermore, the use of specific machine learning and/or deep learning techniques enables the system to continuously improve the accuracy of the estimates it generates, by analysing historical data collected.

In a second aspect thereof, the invention also relates to an apparatus for monitoring wine in a bottle during a respective cold chain. The apparatus preferably comprises a monitoring device, which in turn comprises a main body configured to be applied to the bottle. Preferably, the monitoring device comprises a sensor group with sampling frequencies that are preferably adjustable, configured to detect a plurality of predefined physical parameters relating to the wine in the bottle, thereby generating a data set. This data set preferably comprises a respective time series for each of the predefined physical parameters. In this case too, the predefined physical parameters preferably comprise at least two of the following: temperature, humidity and the spatial orientation of the bottle containing the wine. It is also preferable that the predefined physical parameters comprise all the aforementioned parameters relating to the temperature, humidity and spatial orientation of the aforementioned bottle containing wine.

Preferably, the apparatus comprises a distributed computing environment which, preferably, in turn comprises a first artificial intelligence model. The aforesaid first artificial intelligence model is preferably trained to generate a quality index representative of storage conditions of the wine in the bottle as a function of predefined physical parameters. Preferably, the quality index identifies conditions that could compromise one or more qualitative and/or quantitative parameters of the wine in the bottle.

Preferably, the apparatus comprises a user interface configured to provide a user with the quality index following a generation thereof by the first artificial intelligence model, preferably trained as described above.

Preferably, the user interface is accessible via a web page and/or an application. It should be noted that, in the context of the present invention, the term "application" preferably refers to a computer program configured to run on mobile and/or fixed devices, which enables information to be provided to a user and, where appropriate, instructions to be received from the user.

It is also preferable for the monitoring device to be configured to send the data set. Furthermore, it is preferable for the distributed computing environment to be configured to receive this data set.

The apparatus, constructed and configured in this way, enables the advantages previously discussed for the method according to the first aspect of the invention to be achieved. In fact, the monitoring device applicable to the bottle allows predefined physical parameters to be detected directly on the bottle itself, thereby providing more reliable readings. The use of a suitably trained artificial intelligence model makes it possible to estimate, effectively and reliably, the quality index relating to the storage conditions of wine during the respective cold chain. As previously noted, the artificial intelligence model makes it possible to overcome, or at least mitigate, the problem associated with the complexity of the correlations linking predefined physical parameters to the qualitative and/or quantitative parameters of the wine in the bottle.

A third aspect thereof, the invention also relates to a software product for monitoring wine in a bottle during a respective cold chain. The software product comprises instructions which, when executed by an apparatus in accordance with the second aspect of the invention, cause one or more of the steps, preferably all the steps, of a method according to the first aspect of the invention to be performed.

In a further aspect thereof, the invention also relates to a computer-readable medium containing a software product in accordance with the third aspect of the invention.

The present invention, in one or more of the foregoing aspects, may also exhibit one or more of the following preferred features.

In some embodiments, the step of the method for generating the quality index comprises in turn sub-steps. A first sub-step preferably provides for defining a respective optimum range of values for each of the predefined physical parameters detected.

Preferably, the first sub-step also involves, for each of the predefined physical parameters detected, evaluating whether the respective time series falls outside the respective optimum range of values and, if so, for how long.

Preferably, the first sub-step also involves, for each of the predefined physical parameters detected, associating a respective weight variable as a function of the possible time spent by the respective time series outside the respective optimum range.

Preferably, the first sub-step also involves, for each of the predefined physical parameters detected, estimating a respective hazard index as a function of the deviation of the respective time series from the respective optimum range of values. Preferably, the first sub-step involves weighting the respective hazard index as a function of the respective weight variable.

It is also preferable for a second sub-step to comprise estimating the quality index based on the weighted hazard index associated with at least one of the predefined physical parameters. Alternatively, it is preferable to estimate the quality index as a function of one or more of the respective weighted hazard indices, preferably as a function of each of the respective weighted hazard indices associated with the predefined physical parameters detected.

These sub-steps define an algorithm for generating the quality index, which proves to be particularly effective and reliable when implemented by an artificial intelligence model trained in accordance with the first aspect of the invention. When properly trained, the artificial intelligence model enables the precise identification and selection of optimum ranges and provides a reliable estimate of the hazard indices associated with deviations from these ranges. Furthermore, the ability of the trained artificial intelligence model to process non-linear relationships and complex interdependencies between variables makes it possible to estimate the final environmental quality index even in the presence of multiple interconnected environmental factors. More generally, an artificial intelligence model specifically trained in accordance with the above, combined with the quality index evaluation algorithm mentioned above, enables the creation of an adaptive and robust system capable of refining the critical thresholds and weights of the hazard indices over time. The artificial intelligence model therefore makes the quality index evaluation algorithm dynamic and scalable across different types of wine and their respective storage conditions.

In some embodiments, the method comprises sending automatic notifications and/or alarms, preferably both, to the user in order to report anomalies and/or to allow interventions on the bottle of wine. This enables prompt action to be taken regarding the wine's storage conditions should any issues be detected, thereby preventing - or at the very least reducing - any potential damage to the wine being monitored.

In some embodiments, the method comprises providing the user with at least one of the respective time series, preferably all the respective time series for the predefined physical parameters detected, in the form of a report, such as a weekly, monthly or annual graph. This enables the user to verify the storage conditions to which the wine has been subjected throughout the respective cold chain, thereby certifying - or at least ensuring with reasonable certainty - that the quality of the wine in question has remained unchanged. This certification is particularly important for enhancing the market value of the monitored product. At public auctions for the sale of lots of fine wine, the value of the product is, in fact, influenced by a number of factors, including traceability and certification of storage conditions. When a batch of bottles is accompanied by a cold chain certificate, usually issued by the producer to guarantee optimal storage conditions within their own cellar, the value of the wine tends to increase significantly. This certification provides reasonable assurance that the wine has been kept under essentially ideal conditions throughout the entire storage period, avoiding, for example, sudden temperature fluctuations or unsuitable humidity levels that could compromise its quality. From a market perspective, the presence of an official certification attesting to the correct maintenance of storage conditions can result in an increase in the value of the batch of up to 30% compared with bottles without such a guarantee. This is because buyers and collectors, aware of the importance of storage conditions, tend to place a premium on wines for which impeccable storage can be reasonably demonstrated.

In some embodiments, it is preferable for the method to comprise at least one of the following: sending automatic notifications and/or alarms to the user in order to report anomalies and/or to allow interventions on the bottle of wine, and providing the user with at least one of the respective time series in the form of a report.

In some embodiments, the apparatus comprises a mobile device on which an application is running that enables interaction with the user interface. This allows the user to receive the quality index and any further information about the wine in the bottle, wherever they are.

In some embodiments of the method and/or the apparatus, it is preferable that the predefined physical parameters also comprise one or more of the following: acceleration, the intensity of light and/or ultraviolet radiation incident on the wine bottle, the fill level of the bottle and the integrity of the bottle, preferably understood as the structural integrity of the bottle and preferably evaluated by measuring a change in the fill level over time. These are all other physical factors that could affect the storage condition of the wine in the bottle, altering its qualitative and/or quantitative characteristics. Monitoring one or more of these additional parameters can improve the reliability of the hazard evaluation of the storage conditions.

In some embodiments, the apparatus comprises a gateway device, preferably configured to put the monitoring device and the distributed computing environment in mutual data communication.

It should be noted that, in the context of the present invention, the term "gateway device" is preferably understood to mean a hardware support that acts as an access point and intermediary between two networks, preferably with different communication protocols, enabling them to interact.

In some embodiments, the step of supplying the input data set to the first trained artificial intelligence model involves placing the gateway device in data connection with the monitoring device and the distributed computing environment that comprises the first artificial intelligence model. Preferably, this also comprises sending the data set from the monitoring device to the gateway device. Preferably, this also comprises sending the data set from the gateway device to the distributed computing environment.

In some embodiments of the method and/or the apparatus, the sensor group comprises an accelerometer or a gyroscope, preferably both, which are configured to detect movements and/or oscillations of the monitoring device comprising the sensor group. It is also preferable that the step of supplying the input data set to the first trained artificial intelligence model comprises the sub-step of suspending the transmission of the data set from the monitoring device to the gateway device, whenever the accelerometer or gyroscope detects movements and/or oscillations of the monitoring device exceeding a predetermined threshold value.

This predetermined threshold value, in terms of acceleration, is, for example, preferably comprised between 0.2 m/s² and 2.5 m/s², and even more preferably equal to 1.0 m/s² ± 0.3 m/s². This makes it possible to identify, preferably automatically, the transport status of the monitoring device applied to the wine bottle, thereby activating an energy-saving operating mode.

During such transport, the monitoring device is often unable to establish a stable connection with the distributed computing environment or with the data collection gateway. Therefore, attempting to transmit the detected environmental data would result in potentially unnecessary energy consumption.

Transmission of the data set is preferably resumed automatically when the accelerometer detects a period of stability, i.e. a prolonged absence of significant changes in acceleration and/or oscillation frequency.

For example, a steady-state condition can be identified when changes in acceleration remain below 0.05 m/s² for a continuous period of at least 10 minutes, or when the measured oscillation frequency is less than 0.2 Hz over the same time interval.

Given that the predefined physical parameters define the respective variation frequencies, in some embodiments of the method and/or the apparatus, the first artificial intelligence model is further trained to regulate the sampling frequencies of the sensor group by increasing and/or decreasing them in line with respective increases and/or decreases in the variation frequencies of the predefined physical parameters. Consequently, it is preferable that the step of detecting the predefined physical parameters involves using the first artificial intelligence model, which has been further trained to regulate the sampling frequencies of the sensor group in accordance with the variation frequencies of the predefined physical parameters.

Additionally or alternatively, the distributed computing environment may comprise a second artificial intelligence model trained to regulate the sampling frequencies of the sensor group as a function of the variation frequencies of predefined physical parameters.

Consequently, the step of the method involving the detection of predefined physical parameters preferably comprises using this second artificial intelligence model to regulate the sampling frequencies as described above.

This improves the energy efficiency of the monitoring devices by regulating the sampling frequencies of the sensor group to match the frequencies at which the predefined physical parameters to be detected change, thereby adapting energy consumption to operating conditions. In particular, the use of an artificial intelligence model such as the one described above offers the advantage of effectively adapting the sensor group's operation to changes in the environmental conditions to which the bottle is subjected. In fact, adjusting the sampling frequency to match the variation frequency of the parameters being monitored makes it possible to reduce the overall energy consumption of the sensors themselves.

In some embodiments of the method and/or the apparatus, the first artificial intelligence model is further trained to estimate current and/or future values of the qualitative and/or quantitative parameters of the wine in the bottle, preferably based on time series for predefined physical parameters. Consequently, the method preferably comprises using the first artificial intelligence model, trained in this way, to estimate current and/or future values of the wine's qualitative and/or quantitative parameters as a function of the time series.

Additionally or alternatively, the distributed computing environment may comprise a third artificial intelligence model trained to estimate current and/or future values of the qualitative and/or quantitative parameters of the wine as a function of the time series.

Consequently, it is preferable for the method to comprise the step of using the third artificial intelligence model, trained as described above, to estimate current and/or future values of the qualitative and/or quantitative parameters of the wine, as described above.

Advantageously, the method thus makes it possible to predict the temporal evolution of the parameters of interest with reasonable accuracy.

More generally, it is preferable for the distributed computing environment to comprise at least one of the following: a second artificial intelligence model trained to regulate the sampling frequencies of the sensor group, increasing and/or decreasing them in line with respective increases and/or decreases in the variation frequencies of the predefined physical parameters, and a third artificial intelligence model trained to estimate current and/or future values of the qualitative and/or quantitative parameters of the wine in the bottle as a function of the respective time series for each of the predefined physical parameters.

In some embodiments, the apparatus comprises three radio beacons, preferably not aligned with one another, each configured to emit a respective signal. The sensor group is preferably further configured to detect the respective signal intensities for each of the respective signals emitted by the three radio beacons. Preferably, the monitoring device is further configured to transmit the respective signal intensities, and the distributed computing environment is preferably further configured to receive these respective signal intensities. It is also preferable for the distributed computing environment to comprise a trilateration algorithm for computing a position of the monitoring device as a function of the respective signal intensities. Preferably, the user interface is further configured to provide the calculated position of the monitoring device to the user.

In some embodiments, the method comprises the step of arranging the three radio beacons, each emitting a respective signal, in such a way that they are not aligned with one another. Preferably, the method also comprises the step of detecting, by the sensor group, respective signal intensities for each of the respective signals emitted by the three radio beacons. It is then preferable to calculate the position of the monitoring device as a function of the respective signal intensities. It is also preferable for the method to include a step of providing the user with the location of the monitoring device as calculated. This not only makes it easier to locate a bottle of wine within the cellar during the sales process, but also simplifies any action the user may need to take regarding the bottle. In fact, being able to quickly and easily identify one or more bottles of wine, for example, when they are subjected to storage conditions that the artificial intelligence model deems hazardous, makes it possible to take prompt action regarding those bottles in order to prevent, or at least reduce, the risk of damage, including permanent damage, to the wine in those bottles.

In some embodiments of a computer-implemented method, in accordance with the first aspect of the invention, which uses an apparatus in accordance with the second aspect of the invention, the method comprises the step of detecting data relating to the energy consumption of the monitoring device and/or other components of the apparatus. Preferably, this method also comprises analysing energy consumption data using an artificial intelligence model that has preferably been trained to perform at least one of the sub-steps of defining strategies for optimizing energy consumption and/or recognizing abnormal energy consumption conditions. This artificial intelligence model may be any one of the first, second or third artificial intelligence models mentioned earlier, or it may be a further artificial intelligence model that has been specifically trained.

Preferably, the sub-step of defining energy consumption optimization strategies comprises automatically regulating the sampling frequencies of the sensors and/or modulating communication between the components of the apparatus, in order to extend the operational autonomy of the monitoring device. As described above, this makes the monitoring device more energy-efficient; the device is usually battery-powered, and its battery life naturally depends on its energy efficiency. Improving this energy efficiency consequently helps to extend the battery life, thereby reducing the number of times the monitoring device for a specific bottle of wine needs to be replaced.

Preferably, the step of identifying abnormal energy consumption involves generating a corresponding warning and subsequently notifying the user of this warning, so as to prompt the user to take immediate action to rectify the abnormal energy consumption.

This helps to continuously improve the energy efficiency of the monitoring device, enabling the timely identification and resolution of abnormal consumption conditions that could affect the monitoring device's lifespan.

Preferably, the step of analysing energy consumption data using an artificial intelligence model also comprises sending subsequent warnings if, despite corrective action, abnormal energy consumption persists, preferably indicating that, unless quality parameters and/or optimal operating conditions are restored, battery life may be compromised.

### DETAILED DESCRIPTION OF PREFERRED EMBODIMENTS

The features described above, as well as further features and the advantages of the invention, will be better understood from the detailed description of certain preferred embodiments illustrated, by way of non-limiting example, with reference to the accompanying drawings, in which:
- Figure 1 is a schematic view of a monitoring device according to a first embodiment of the invention, applied to a bottle containing wine;
- Figure 2 is a schematic exploded side view of the monitoring device shown in Figure 1;
- Figure 3 is a schematic view showing an apparatus for monitoring bottled wine in accordance with a first embodiment of the present invention, in which the main data communications between the various components of the apparatus are shown;
- Figure 4 is a schematic diagram showing a wine monitoring apparatus in accordance with a second embodiment of the present invention;
- Figure 5 is a block diagram illustrating a preferred embodiment of a computer-implemented method for monitoring bottled wine, according to the present invention.

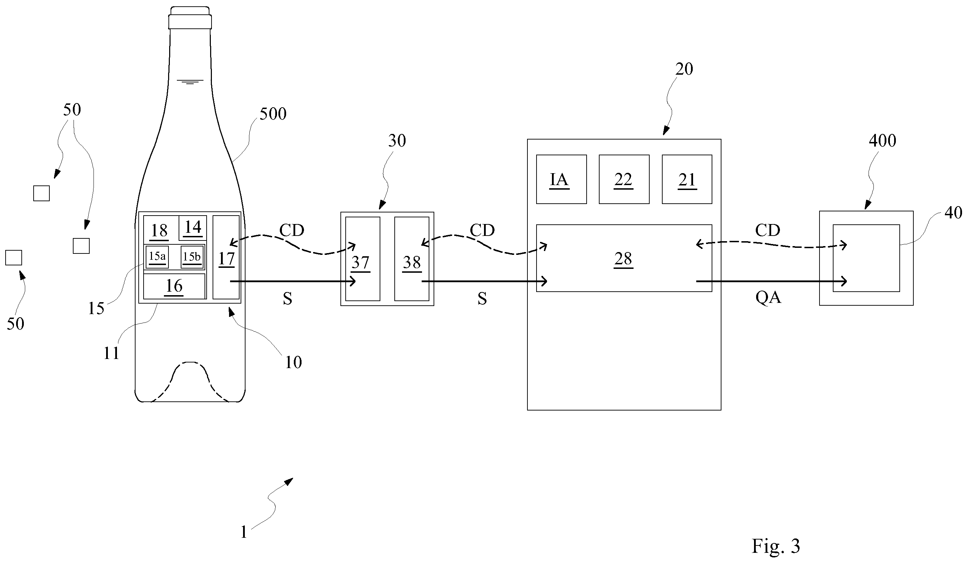

An apparatus for monitoring wine in a bottle, designated by reference number 500, during a respective cold chain is generally designated by reference number 1. As shown in Figure 3, the wine monitoring apparatus 1 comprises a monitoring device 10, a distributed computing environment 20, a gateway device 30, a user interface 40 and three non-aligned radio beacons 50, where each of the three radio beacons 50 is configured to transmit a signal, preferably of a type commercially known as BLE, i.e. Bluetooth^{®} Low Energy. The various components of the apparatus 1 for monitoring wine will be described in more detail below.

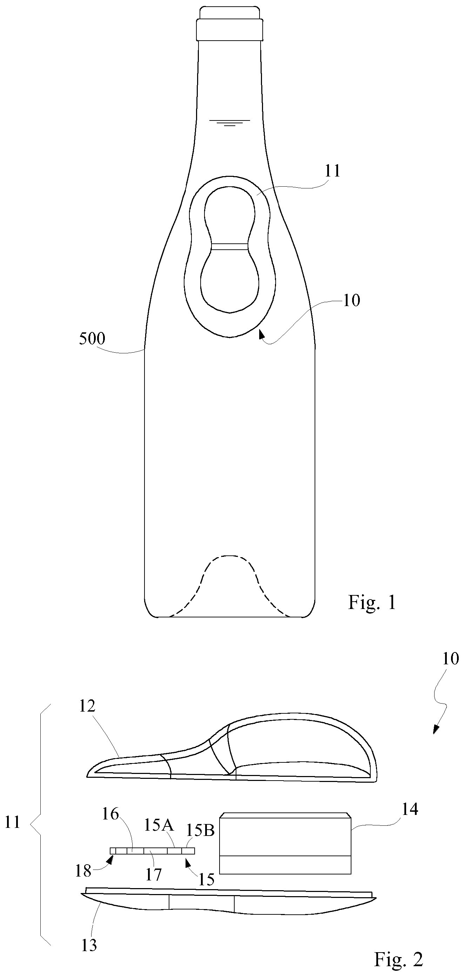

With reference to Figure 1, the monitoring device 10 comprises a main body 11 designed to be applied to the bottle 500, for example by means of a double-sided adhesive tape specifically designed to adhere to glass surfaces, or by shape coupling, or by means of any mechanical retaining elements securing the main body 11 to the bottle 500.

Considering now Figure 2, which shows a schematic exploded side view of the monitoring device 10, it is observed that the main body 11 is formed by joining two distinct, opposing parts 12 and 13. The monitoring device 10 further comprises a sensor group 15, in turn comprising a plurality of sensors, each having respective adjustable sampling frequencies.

The sensor group 15 is configured to detect a plurality of predefined physical parameters relating to the wine in a bottle 500, thereby obtaining a data set S comprising a time series for each of the predefined physical parameters detected. The predefined physical parameters comprise at least two parameters from temperature, humidity and spatial orientation of the bottle 500. However, it is advantageous for these physical parameters to also comprise one or more of acceleration, integrity of bottle 500, fill level, and intensity of the light and/or ultraviolet radiation incident on the bottle 500.

These are all factors that may, in some way, contribute to changes in the qualitative and/or quantitative parameters representative of the actual quality of the wine being monitored. For example, excessively high or low temperatures can accelerate chemical reactions in wine, including oxidation, which can alter the concentration of certain molecules such as polyphenols. Oxidation can cause wine to lose its freshness, aroma and colour. Such temperatures can also accelerate the wine's ageing process and adversely affect its balance and complexity, leading to a loss of structure and harmony. The volatile aromas present in wine can be compromised by such temperatures, resulting in a loss of intensity and aromatic complexity. High temperatures can also cause the wine to expand within the bottle, increasing the internal pressure and compromising the seal of the cork. This can allow oxygen to enter the wine, accelerating oxidation and compromising its chemical stability. High temperatures also create an environment conducive to the proliferation of microorganisms in wine, including bacteria and yeasts. This increases the risk of microbiological contamination, which can lead to sensory defects or even the complete spoilage of the wine.

Even excessively low humidity levels can cause air to seep into the bottle, as the cork tends to shrink as it dries out. On the contrary, excessive humidity levels can encourage the growth of mould and bacteria.

Furthermore, the angle at which the bottle is held can also affect the seal provided by the cork. In fact, it is generally preferable to store wine bottles lying down, so that the wine, by wetting the cork, keeps it sufficiently moist to prevent it from drying out.

The accelerations to which the bottle is subjected, as well as being important indicators of any impacts it may have suffered, can cause the wine's sediment to be stirred up unintentionally. Obviously, the integrity of the bottle is essential to prevent the contents from leaking out or air from entering the bottle; the fill level of the bottle is also a useful indicator of conditions that could be detrimental to the wine's preservation.

The intensity of the light radiation falling on the bottle can lead to various changes in the qualitative and/or quantitative parameters representative of the quality of the wine. In particular, sunlight, or artificial light with a high ultraviolet component, can, if too intense, alter the aromatic molecules in wine, causing a change in its flavour. This phenomenon is particularly pronounced in white and sparkling wines. Furthermore, light can break down the molecules responsible for pigmentation.

The qualitative and/or quantitative parameters indicative of wine quality may vary, each being related to changes over time in one or more of the predefined physical parameters listed above. For this reason, it is not generally possible to define a simple mathematical relationship that describes these correlations and allows for the easy quantification of any potential risks associated with the environmental conditions in which wine is stored, or that enables the reliable prediction of trends in these qualitative and/or quantitative parameters.

Referring again to Figure 2, the monitoring device 10 also comprises a memory unit 16 configured to store the data set S detected by the sensor group 15. The monitoring device 10 also comprises a first short-range transceiver 17 configured to be able to transmit the data set S. This transceiver may, for example, also exploit the technology commercially known as BLE.

It may also be noted that the monitoring device 10 comprises a control unit 18, preferably comprising a microprocessor, configured to regulate and manage the operation of the sensor group 15 and the other components of the monitoring device 10.

Overall, in the illustrative embodiments described here, the various components of the monitoring device 10 are powered by a battery 14, which is preferably designed to be replaceable. In some embodiments not shown, the battery 14 may be of the rechargeable type. Advantageously, the sensor group 15 also comprises operational sensors 15a designed to detect data relating to the overall energy consumption of the monitoring device 10, or otherwise configured to detect operational data, such as the remaining charge level or the voltage of the battery 14. The sensor group 15 also advantageously comprises positional sensors 15b adapted to detect positional data relating to the monitoring device 10 and, consequently, to the bottle 500 to which it is applied. Such position sensors 15b may, for example, comprise an accelerometer and/or a gyroscope. Given that each of the three radio beacons 50 is configured to transmit a respective signal, for example of the BLE type, the positional sensors 15b also comprise at least one sensor adapted to detect a respective signal intensity for each of the respective signals emitted by the three radio beacons 50.

As shown in Figure 3, the distributed computing environment 20 comprises first long-range transceiver units 28 configured to receive the data set S, or a processed version thereof. Long-range transceiver systems 28 can exploit the internet for receiving and transmitting data, and may comprise antennas for wireless transmission and/or wired systems that use, for example, fibre-optic cables.

The distributed computing environment 20 comprises a respective memory unit 21 configured to store the information it receives and/or processes.

The distributed computing environment 20 also comprises a first artificial intelligence model, indicated in the figure by the abbreviation IA. This artificial intelligence model IA is trained to generate a quality index, referred to as QA, based on predefined physical parameters. The quality index QA can reflect the storage conditions of the wine in the bottle and may, for example, consist of a numerical rating whose value indicates the extent to which the storage conditions in question could compromise, either reversibly or irreversibly, one or more of the qualitative and/or quantitative parameters of the wine being monitored. In other words, the quality index can therefore provide an indication of the events that occurred during storage. In this way, the quality index can help both consumers and retailers to obtain at least an estimate of the wine's condition; whilst this estimate cannot provide absolute guarantees, it does offer a useful guide. More generally, the quality index QA is thus designed to identify environmental conditions that are potentially sub-optimal and/or unsuitable for the proper storage of the wine being monitored, which could affect the quality of the wine, whilst preferably not providing a direct evaluation of the condition of the wine inside the bottle.

The first artificial intelligence model IA can utilize various automatic learning techniques, also known as machine learning, such as, for example, deep neural networks, regression algorithms, decision trees or other well-known techniques. For the classification of wine storage conditions based on predefined physical parameters, that is, for the generation of the quality index QA, the use of recurrent neural network algorithms, also known by the acronym LSTM, or of algorithms generally known as random forests, may be particularly advantageous; however, the use of other well-known algorithms is also possible for the implementation of the present invention.

Overall, a preferred training strategy for generating the quality index QA is supervised learning, which involves feeding a labelled data set into an artificial intelligence model, i.e. data that has already been classified or associated with an output category, which may also be identified by a numerical value. The model is designed to identify schemas, also known as patterns, in the data, enabling it to map each input data point to the respective class or output value. This type of learning enables the model to make predictions or classifications that are reasonably accurate.

In the context of the present invention, supervised learning for the first artificial intelligence model IA can be carried out using historical data of predefined physical parameters, collected by sensors in real-world situations and/or controlled environments. This data can then be labelled with the assistance of expert oenologists and/or through laboratory tests carried out directly on the wine being monitored, in order to determine the final condition of the product in relation to the measurements taken. The labelling process is therefore based on expert knowledge and/or test results, which make it possible to link the final condition of the product to each data set, defined in terms of possible variations in the qualitative and/or quantitative parameters as described above.

The following describes, by way of non-limiting example, a preferred procedure for obtaining labelled data suitable for training the artificial intelligence model IA effectively, so that it generates a quality index QA with a reasonably high level of accuracy.

The first step is to take an initial sample, for example by selecting one hundred bottles from the same batch, that is, bottles with virtually identical, or at least very similar, oenological characteristics. A first objective experimental measurement is then carried out of the qualitative and/or quantitative parameters associated with these bottles, in order to establish a uniform baseline value.

The initial sample is then preferably divided into separate groups, each of which is subjected to different environmental storage conditions for a predetermined exposure period, for example a few hours or a few days. It is preferable that the different storage conditions to which the various groups are subjected simulate predefined physical parameters typical of possible storage and/or transport scenarios.

For example, the initial sample of one hundred bottles can be divided into five groups of twenty bottles, each subjected to different conditions for the same predefined exposure time.

The first group, for example, should be stored under optimal - or near-optimal - conditions, preferably with the temperature comprised between 10°C and 25°C, humidity between 55% and 85% RH, and each bottle kept in a horizontal position.

The second group may, for example, be exposed to temperatures that are slightly higher than the optimal range, such as around 35°C. Humidity levels and the position of the bottles may vary; for example, the same ideal humidity level can be maintained whilst storing the bottle upright, or the humidity can be adjusted whilst keeping the bottle in a horizontal position.

The third group may, for example, be subjected to very high temperatures, preferably around 45°C, with possible variations in humidity, position and/or other predefined physical parameters, so as to simulate even more stressful conditions for the bottles of wine being monitored.

The fourth group, for example, may be exposed to low temperatures, preferably around 5°C. Here too, it is possible to test various humidity conditions, positions and/or other predefined physical parameters.

The fifth group may, for example, be subjected to extremely low temperatures, close to around 0°C, whilst possibly varying the other predefined physical parameters.

It is to be noted that it is possible to select a different number of initial bottles for sampling, as well as different groupings and the environmental conditions to which they are subjected.

Periodically, each bottle in every group undergoes objective, laboratory-based measurements of the qualitative and/or quantitative parameters of the wine it contains, so as to record how the wine reacts to each change in the predefined physical parameters to which it is subjected. It is also possible to assign - even manually - a specific quality index QA to each environmental condition experienced by a particular group or a particular bottle, reflecting how that environmental condition has affected the aforementioned qualitative and/or quantitative parameters.

The experimental procedure described above makes it possible to collect reasonably comprehensive, diverse and objective labelled data, which can be effectively used to train the model IA.

The experimentally labelled data obtained can then be used to train the first artificial intelligence model IA using supervised learning algorithms, such as the aforementioned random forest. The aim is therefore to teach the model IA how storage conditions (identified by the respective time series of predefined physical parameters) can influence the final quality of the wine (identified by qualitative and/or quantitative parameters).

In this way, the first model IA is able to analyse new, previously unlabelled data and make estimates and/or predictions, identifying potential risks or favourable conditions in real time, based on past, current and/or forecast conditions. The model IA is thus able to generate a quality index QA that can reasonably be linked to any potential risks associated with the storage conditions of the monitored wine, following receipt of the data set S collected by the sensor group 15 of the monitoring device 10.

Referring to Figure 3, it is noted that the long-range transceiver units 28 are also configured to send the quality index QA generated by the model IA to the user interface 40, so that it can be presented to a user.

Advantageously, the model IA is further trained to estimate current and/or future values of the qualitative and/or quantitative parameters of the wine being monitored, based on the respective time series for each of the predefined physical parameters. This training can be carried out using the same strategy described above; indeed, by feeding the model IA with labelled data as described above, in particular by associating the final characteristics of the wine in terms of qualitative and/or quantitative parameters with each package of historical data, it is possible to train the model IA to estimate these parameters based on the detected time series and, where appropriate, based on the initial values of the aforementioned qualitative and/or quantitative parameters. This is particularly useful for predicting, with reasonable confidence, the point at which the wine might undergo irreversible deterioration and taking preventive action to avoid it.

In certain embodiments not shown, in addition to or as an alternative to this further training of the model IA, the distributed computing environment may include a second artificial intelligence model trained in any of the ways described above.

Given that the predefined physical parameters define respective variation frequencies, it is also advantageous for the first artificial intelligence model IA to be trained to regulate the sampling frequencies of the sensor group 15 increasing and/or decreasing them in line with the respective increases and/or decreases in the variation frequencies of the predefined physical parameters. Adjusting the operating frequencies in line with operating conditions improves the overall energy efficiency of the monitoring device 10, thereby increasing, or at least substantially optimizing, the life of the battery 14. It is also preferable that, in order to improve the energy efficiency of the overall system, the first model IA should be trained to adjust the communication functions of the monitoring device 10 as well. In other words, it is advantageous for the model IA to control both the sampling frequency of the sensor group 15 and the transmission frequency by the device 10 of the data set S detected by that sensor group 15.

Here too, training can be carried out using various strategies; however, the inventor has found a training strategy based on reinforcing the model IA to be particularly effective.

In the context of the present invention, reinforcement learning is used to improve the management of the sampling frequency and/or transmission frequency of the data set S from the sensors of the sensor group 15, with the aim of reducing their energy consumption. In this process, the sampling frequency of the parameters to be detected is dynamically regulated in response to variations in those parameters. When the monitored parameters change more rapidly, the sampling frequency increases to capture more detailed data; conversely, if changes are less frequent, the sampling frequency is reduced to save energy.

The reinforcement learning system enables the model IA to optimize these parameters in real time, rewarding actions that lead to efficient use of resources and penalizing those that result in energy wastage, so that the system gradually learns to balance data accuracy with energy efficiency.

In certain embodiments not shown, in addition to or as an alternative to this further training of the model IA, the distributed computing environment may include a separate third artificial intelligence model trained as described above.

It is also advantageous for the distributed computing environment 20 to include a trilateration algorithm for calculating the position of the monitoring device 10, executed by the first artificial intelligence model IA or by a separate processing unit not shown in the figure.

The trilateration algorithm makes it possible to calculate the position of the monitoring device 10 with respect to the three radio beacons 50, based on the respective signal intensities of each of the respective signals emitted by the radio beacons 50, measured by the appropriate sensors comprised in the positional sensors 15b and shown schematically in Figure 2. Consequently, it is advantageous for the monitoring device 10 to be configured to transmit the respective signal intensities, preferably via the transceiver 17, and for the distributed computing environment 20 to be configured to receive these signal intensities, preferably via the first transceiver means 28.

Figure 3 also shows that the gateway device 30 comprises a second short-range transceiver 37 and second long-range transceiver means 38. The second transceiver 37 is preferably of the BLE type and is configured to exchange data with the first transceiver 17 of the monitoring device 10. The second long-range transceiver means 38, on the other hand, are configured to exchange data with the first long-range transceiver means 28. Like the latter, in fact, the second long-range transceiver means 38 enable the gateway device 30 to exploit the internet for receiving and sending data, and may comprise antennas for wireless transmission and reception and/or wired systems employing, for example, fibre-optic cables.

Overall, the gateway device 30 is designed to enable data communication between the sensor device 10 and the distributed computing environment 20, thereby facilitating a continuous exchange of information between the two. The use of this intermediate component also helps to improve the life of the battery 14 of the monitoring device 10. In fact, if the monitoring device 10 were to communicate directly with the distributed computing environment 20, it would require some form of long-range transceiver, which typically consumes significantly more energy than short-range transceivers. Conversely, gateway devices are generally powered by standard AC power sources, such as domestic or industrial mains sockets, and can therefore easily utilize both short-range transceivers and long-range transceiver means.

It is noted that in the figures the reference 'CD' indicates data communication between components of the apparatus 1.

The gateway device 30 is also designed to decode the data it receives from the monitoring device 10 and, where necessary, convert it into other formats that can be read by the distributed computing environment 20, such as the format commercially known as JSON.

The gateway device 30 is also advantageously configured to work in conjunction with a broker programme 22 comprised in the distributed computing environment, which may, for example, use an MQTT protocol to route the information that needs to be transmitted between the various components of the wine monitoring apparatus 1.

Referring again to Figure 3, it can be seen that, in the example shown, the user interface 40 is accessible to the user via an application running on a mobile device 400, which is shown only schematically. However, this application can also be run on one or more different devices, such as desktop computers or other computing hardware.

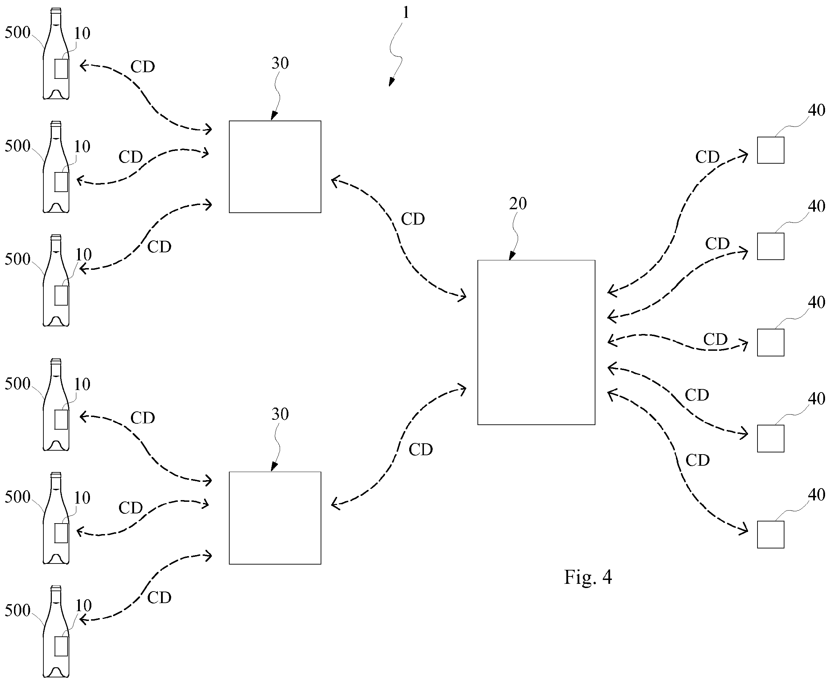

A wine monitoring apparatus such as the one described above can also be used, mutatis mutandis, to monitor a plurality of separate bottles of wine more or less simultaneously. With reference to Figure 4, features similar and/or equivalent to those described above have been designated with the same reference numbers for the sake of simplicity and clarity.

The figure shows the apparatus 1 for monitoring a plurality of wine bottles, each marked with the reference number 500. The apparatus 1 comprises a plurality of monitoring devices 10 defining a monitoring assembly 50; each of the devices 10 has one or more of the features described above and is configured to be applied to one of the bottles 500 respectively. In some embodiments, the apparatus 1 comprises a plurality of monitoring assemblies 50.

The apparatus 1 also comprises a plurality of gateway devices 30, each of which is associated with a respective monitoring assembly 50. The gateway devices 30 are configured to connect the devices 10 of the respective monitoring assembly 50 to the distributed computing environment 20, which has characteristics as described above.

Furthermore, the distributed computing environment 20 is configured to communicate with one or more applications 40; in particular, it is configured to transmit information to, and/or receive information from, the aforementioned one or more applications 40, which is transmitted by a user interacting with it.

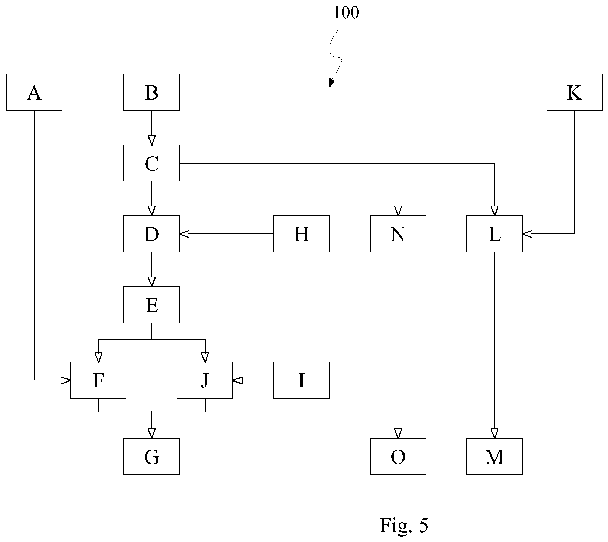

Referring now to Figure 5, this shows a block diagram of the steps of a preferred embodiment of a computer-implemented method, denoted by reference number 100, for monitoring bottled wine during a respective cold chain. The method 100 utilizes the apparatus 1 in accordance with the description given so far; consequently, for a detailed description of the steps of the method 100, reference is to be made to the components of the apparatus 1 mentioned earlier. However, the steps described below may also be implemented, mutatis mutandis, by means of other embodiments of the apparatus according to the present invention.

It should be noted that the steps of the aforementioned method 100 are presented in an order selected for the sake of clarity; however, a person skilled in the art would be able to identify further different combinations and/or orders of implementation of all or only some of the aforementioned steps, whilst remaining within the scope of protection defined by the appended claims.

The method 100 comprises a step A, which involves training the first artificial intelligence model IA to generate the quality index QA based on two or more predefined physical parameters. This training preferably takes place in accordance with the procedure described above.

The method 100 also comprises a step B of providing the monitoring device 10, which includes the sensor group 15 with adjustable sampling frequencies. Next, in step C, the device 10 is applied to the bottle 500 containing the wine to be monitored.

The method 100 also comprises a step D in which the predefined physical parameters relating to the wine in the bottle 500 are detected using the sensor group 15, thereby obtaining the data set S, which comprises a respective time series for each of the physical parameters.

Advantageously, the method 100 comprises a step E of providing the model IA with the data set S, and a step F of generating the quality index QA based on the data set S using the model IA trained according to step A.

With regard to the components of the apparatus 1, the aforementioned step E of the method 100 advantageously comprises a plurality of sub-steps. An initial sub-step of step E consists of establishing a data connection between the gateway device 30 and the monitoring device 10, as well as with the distributed computing environment 20, which comprises the first artificial intelligence model IA. A second sub-step consists of sending the data set S from the monitoring device 10 to the gateway device 30, whilst a third sub-step consists of sending that data set S from the gateway device 30 to the distributed computing environment 20.

To improve the energy efficiency of the monitoring device 10, it is particularly advantageous for step E to also involve suspending the transmission of the data set S from the device 10 to the gateway device 30 if the accelerometer and/or gyroscope, comprised in the positional sensors 15b, detects (or detect) movements and/or oscillations of the aforementioned device 10 exceeding a predetermined threshold value. In fact, such displacement and/or vibration values could indicate that the bottle 500 is being transported under conditions in which a gateway device for transmitting the data set S is not usually accessible. In the absence of a gateway, attempting to send this data set S would therefore constitute a largely pointless waste of energy. During transport, it is in fact preferable to store the detected data in the memory unit 16. The transmission of the data set S is preferably automatically resumed when the accelerometer and/or gyroscope detects (or detect) a stable condition, i.e. the prolonged absence of significant changes in acceleration and/or oscillation frequency.

The threshold value and stability condition can be selected on the basis of the operating conditions, preferably in accordance with the transport scenario in question. By way of non-limiting example, *Table I* below sets out preferred threshold ranges, both in terms of acceleration and oscillation frequency, for various possible transport scenarios, and defines possible stability conditions for each scenario.

Depending on the displacement and/or acceleration values detected by the position sensors 15b, it is also advantageous to stop, or reduce, the sampling frequency of the predefined physical parameters to improve the energy efficiency of the monitoring device 10 and to avoid, or at least reduce, the risk of false positives in the measurements taken by the sensor group 15.

| *Table I - Threshold value ranges and stability conditions for different transport scenarios*. | | | |
|---|---|---|---|
| **Transport scenario** | **Threshold value range in terms of acceleration [m/s²]** | **Threshold value range in terms of oscillation frequency [Hz]** | **Stability condition** |
| Road Transport | 0.5 - 1.5 | 0.3 - 1.2 | < 0.1 m/s² for 10 minutes |
| Maritime Transport | 0.2 - 0.8 | 0.1 - 0.5 | < 0.05 m/s² for 15 minutes |
| Air Transport | 1.0 - 2.5 | 1.0 - 3.0 | < 0.2 m/s² for 5 minutes |
| Static Warehouse | < 0.1 | < 0.2 | < 0.05 m/s² for 30 minutes |

In order to generate the quality index QA, the inventor has identified it as particularly advantageous for the aforementioned step F to comprise the following sub-steps. A first sub-step involves, for at least one of the physical parameters detected:
- defining a respective optimum range of values;
- evaluating whether the respective time series falls outside the respective optimum range of values and, if so, for how long;
- associating a respective weight variable as a function of the possible time spent by the respective time series outside the respective optimum range;
- estimating a respective hazard index as a function of the deviation of the respective time series from the respective optimum range of values and weighting the respective hazard index as a function of the respective weight variable;

In some embodiments, it is preferable for the aforesaid first sub-step to be carried out for each of the predefined physical parameters detected.

As for the weighting variables, these can be selected according to different criteria. For example, one possible choice is to classify the time spent outside the optimum range into three categories and assign a predefined weight to each, such as:
- a short exceedance, i.e. less than twenty-four hours, to which a weight variable equal to 1 is assigned;
- average exceedance, i.e. between one and twenty days, to which a weight variable of 3 is assigned;
- prolonged exceedance, i.e. lasting more than twenty days, to which a weight variable of 5 is applied.

The hazard index, on the other hand, is preferably determined automatically by the artificial intelligence model IA, based on the training carried out in accordance with Step A.

A second sub-step of Step F involves estimating the quality index QA based on the weighted hazard index (or indices) assessed in the previous sub-step.

There are several possible options for defining the quality index QA; however, it can be particularly advantageous to choose a single numerical value as the quality index QA, as this allows the quality of the environmental conditions to which the bottle 500 of wine is subjected to be represented simply and effectively. Environmental storage conditions are therefore usefully classified according to the index value QA, which can, for example, be defined on a 10-point scale divided into four distinct categories, defined as follows:
1. Levels 1-2: POOR QA: environmental conditions well outside the acceptable range and a high risk of damage to the wine. The user is required to take action as soon as possible;
2. Levels 3-4: AVERAGE QA: the situation is not ideal, but the risk is limited. It is advisable to monitor the situation and take action if necessary.
3. Levels 5-6: OPTIMAL QA; generally good storage parameters, with only minor deviations observed.
4. Levels 7-10: EXCELLENT QA, having been stored in ideal, or near-ideal, and stable conditions.

The method 100 further comprises a step G in which the generated quality index QA is provided to a user. Advantageously, this step comprises sending the user automatic notifications and/or alarms to report anomalies and/or enable action to be taken regarding the bottle of wine. For example, for levels 3 and 4 mentioned above, the user receives notifications advising them to monitor the bottles closely; whereas, for levels 1 and 2 mentioned above, the user receives an urgent alarm prompting immediate action.

Overall, this enables the user to take prompt action in critical situations, thereby avoiding - or at least reducing - the risk of irreversible damage to the wine in the monitored bottle 500.

In addition to or as an alternative to the quality index QA, the method 100 advantageously provides the user with at least one of the respective time series in the form of a report.

Still with reference to Figure 5, it can be seen that the method 100 comprises a step H which involves further training the model IA to regulate the sampling frequencies of the sensor group 15 increasing and/or decreasing them in accordance with respective increases and/or decreases in the variation frequencies of the predefined physical parameters. This training is carried out in accordance with what has already been discussed regarding the apparatus, in accordance with the preferred embodiments of the present invention. Consequently, step D comprises using the model IA trained in this way to regulate the sampling frequencies of the sensor group 15.

It should also be noted that the aforementioned method 100 comprises two steps, I and J. Step I consists of further training the first artificial intelligence model IA to estimate current and/or future values of the qualitative and/or quantitative parameters of the wine in the bottle 500 based on the time series detected, preferably during step D. Here too, this further training is carried out using methods already described in the description of the preferred embodiments of the apparatus according to the invention. Step J therefore involves using the model IA trained in Step I to estimate current and/or future values of the qualitative and/or quantitative parameters of the wine in the bottle 500, as a function of the time series for the predefined physical parameters.

In some embodiments, such as the one shown in Figure 5, the method 100 also comprises steps adapted to provide the user with a position on a virtual map of the bottle 500. In particular, the method 100 comprises a step K of arranging the three radio beacons 50 in such a way that they are not aligned with one another. Next, there is a step L in which the sensor group 15, specifically the position sensors 15b, detects the respective signal intensities for each of the respective signals emitted by the radio beacons 50. Step L also comprises calculating the position of the monitoring device 10 as a function of the respective signal intensities detected. The method 100 also comprises a step M, which consists of providing the user with the calculated position of the monitoring device 10, and consequently of the bottle 500.

In order to improve the overall energy efficiency of the apparatus 1 used to implement the method 100, the inventor has identified it as particularly advantageous for the aforementioned method 100 to also comprise a step N and a step O, as shown in Figure 5 and described below.

Step N involves detecting data, preferably via operational sensors 15a, relating to the energy consumption of the monitoring device 10 and/or other components of the apparatus 1.

Step O, on the other hand, involves analysing the aforementioned energy consumption data using an artificial intelligence model that has been specifically trained to carry out at least one of the two sub-steps of Step O listed below. It should be noted that this trained artificial intelligence model may be the first model IA or an additional model comprised in the computing environment 20.

The first of the two sub-steps of Step O involves defining strategies to optimize energy consumption as a function of the energy consumption data. Optimization strategies may, for example, comprise automatically regulating the sampling frequencies of the sensor group 15 and/or modulating communication between the components of the apparatus, in order to extend the operational life of the battery 14 in the monitoring device 10. This allows the energy consumption of the device 10 to be adapted to the actual operating conditions, significantly improving its battery life without compromising monitoring performance, and therefore without substantially affecting the reliability of the certification of the wine being monitored.

The second of the two sub-steps of Step O involves identifying abnormal energy consumption patterns, generating a corresponding warning and subsequently notifying the user of this warning, so as to prompt the user to take immediate action to rectify any abnormal energy consumption patterns. It is also advisable to send a follow-up warning if, despite corrective action, the abnormal energy consumption persists, indicating that, unless quality and/or operational parameters are restored to a reasonably optimal level, the life of the battery 14 may be compromised.

The training of the artificial intelligence model that enables the implementation of at least one of the two sub-steps of Step O described above may be carried out in a manner similar to that described previously. In particular, the inventor has identified that training the model IA for this purpose using well-known reinforcement learning algorithms is particularly effective, using as a training criterion the optimization of the energy consumption of the monitoring device 10 whilst maintaining the reliability of the data set S detected by the latter.

Overall, with regard to sending notifications and/or alarms to the user to indicate abnormal storage conditions and/or energy consumption, the inventor has found a three-tiered alarm procedure to be particularly effective, structured as follows:
1. warning notification: triggered in the event of minor, temporary deviations from the optimal temperature, humidity and/or position ranges, or in the event of an initial, non-critical increase in energy consumption. The alarm appears as a non-intrusive notification via the user interface, suggesting active monitoring without requiring immediate action;
2. early warning alarm: triggered in the event of significant or persistent exceedances of predefined environmental limits (for example, when the external temperature remains outside the optimal range for more than 12 hours), or in the event of a significant increase in energy consumption. The system sends a visual and/or audible signal via the application and/or user interface, advising the user to check the condition of the bottle and the surrounding environmental conditions directly;
3. critical alarm: triggered in the event of serious and prolonged sub-optimal environmental conditions (e.g. a significant deviation from the optimal temperature range for more than 24 hours, prolonged exposure to direct light or a dangerous abnormal tilt), or in the event of abnormal energy consumption that compromises the correct functioning of the monitoring device 10. This level triggers a high-priority alarm via push notification, automatic email and event logging in memory unit 21 of the distributed computing environment 20, with the aim of immediately implementing corrective measures.

The present invention also relates to a software product for monitoring wine in a bottle during a respective cold chain (not shown), which comprises instructions that, when executed by an apparatus in accordance with the present invention, cause the steps of a method according to the present invention to be carried out.

Furthermore, the present invention also relates to a computer-readable medium, not shown, containing the aforementioned software product.

The invention thus solves the proposed problem while achieving a plurality of advantages. In particular, it enables continuous and reasonably reliable monitoring of bottled wine substantially throughout the entire respective cold chain. The use of one or more artificial intelligence models offers numerous advantages, as mentioned earlier; furthermore, thanks to user feedback, it is possible to continue improving the reliability of the operations, estimates and/or predictions made by these models.

Obviously, a person skilled in the art may, in order to meet specific and contingent application requirements, make further modifications and variants, all falling within the scope of protection as defined by the following claims.

## Claims

1. A computer-implemented method (100) for monitoring wine in a bottle (500) during a respective cold chain, the method comprising:
A. training a first artificial intelligence model (IA) to generate a quality index (QA) representative of storage conditions of the wine in the bottle (500) as a function of a plurality of predefined physical parameters relating to the wine in the bottle (500), the predefined physical parameters comprising at least two of temperature, humidity and spatial orientation of the bottle (500), the quality index (QA) identifying conditions which could compromise one or more qualitative and/or quantitative parameters of the wine in the bottle (500);
B. providing a monitoring device (10) including a sensor group (15) with adjustable sampling frequencies;
C. applying the monitoring device (10) to the bottle (500);
D. detecting the predefined physical parameters relating to the wine in the bottle (500) by means of the sensor group (15) obtaining a data set (S) comprising a respective time series for each of the predefined physical parameters;
E. providing the data set (S) as input to the first artificial intelligence model (IA) trained according to step A;
F. generating the quality index (QA) as a function of the data set (S) by means of the first artificial intelligence model (IA) trained according to step A;
G. providing the quality index (QA) to a user.

2. Computer-implemented method (100) according to claim 1, wherein step F comprises the sub-steps of:
• for at least one of the detected predefined physical parameters:
- defining a respective optimum range of values;
- evaluating whether the respective time series falls outside the respective optimum range of values and, if so, for how long;
- associating a respective weight variable as a function of the possible time spent by the respective time series outside the respective optimum range;
- estimating a respective hazard index as a function of the deviation of the respective time series from the respective optimum range of values and weighting the respective hazard index as a function of the respective weight variable;
• estimating the quality index (QA) as a function of the weighted hazard index.

3. Computer-implemented method (100) according to claim 1 or 2, comprising at least one of:
• sending automatic notifications and/or alarms to the user in order to report anomalies and/or to allow interventions on the bottle (500) of wine;
• providing the user with at least one of the respective time series in the form of a report.

4. Computer-implemented method (100) according to any one of the preceding claims, comprising:
H. further training the first artificial intelligence model (IA) to regulate the sampling frequencies of the sensor group (15) increasing and/or decreasing them in response to respective increases and/or decreases in the variation frequencies of the predefined physical parameters;
and wherein step D comprises using the first artificial intelligence model (IA) trained according to step H to regulate the sampling frequencies of the sensor group (15) as a function of the variation frequencies of the predefined physical parameters.

5. Computer-implemented method (100) according to any one of the preceding claims, comprising:
I. further training the first artificial intelligence model (IA) to estimate current and/or future values of the qualitative and/or quantitative parameters of the wine in the bottle (500) as a function of the time series;
J. using the first artificial intelligence model (IA) trained according to step I to estimate current and/or future values of the qualitative and/or quantitative parameters of the wine in the bottle (500) as a function of the time series.

6. Computer-implemented method (100) according to any one of the preceding claims, wherein step E comprises the sub-steps of:
• placing a gateway device (30) in data communication with the monitoring device (10) and with a distributed computing environment (20) comprising the first artificial intelligence model (IA);
• sending the data set (S) from the monitoring device (10) to the gateway device (30);
• sending the data set (S) from the gateway device (30) to the distributed computing environment (20).
and wherein the sensor group (15) comprises an accelerometer or a gyroscope and wherein step E further comprises suspending the sending of the data set (S) from the monitoring device (10) to the gateway device (30) if the accelerometer or gyroscope detects movements and/or oscillations of the monitoring device (10) greater than a predetermined threshold value.

7. Computer-implemented method (100) according to any one of the preceding claims, comprising:
K. arranging three radio beacons (50) so that they are not aligned with one another, each of the three radio beacons (50) emitting a respective signal;
L. detecting by means of the sensor group (15) respective signal intensities for each of the respective signals of the three radio beacons (50), subsequently calculating a position of the monitoring device (10) as a function of the respective signal intensities;
M. providing the position of the monitoring device (10) to the user.

8. An apparatus (1; 1') for monitoring wine in a bottle (500) during a respective cold chain, comprising:
• A monitoring device (10) in turn comprising a main body (11) configured to be applied to the bottle (500) and a sensor group (15) with adjustable sampling frequencies configured to detect a plurality of predefined physical parameters relating to the wine in the bottle (500) obtaining a data set (S) comprising a respective time series for each of the predefined physical parameters, the predefined physical parameters comprising at least two of temperature, humidity and spatial orientation of the bottle (500) containing wine;
• A distributed computing environment (20) comprising a first artificial intelligence model (IA) trained to generate a quality index (QA) representative of storage conditions of the wine in the bottle (500) as a function of the predefined physical parameters, the quality index (QA) identifying conditions that could compromise one or more qualitative and/or quantitative parameters of the wine in the bottle (500);
• A user interface (40) configured to provide a user with the quality index (QA) following a generation thereof by the first artificial intelligence model (IA);
wherein the monitoring device (10) is configured to send the data set (S) and the distributed computing environment (20) is configured to receive the data set (S).

9. An apparatus (1; 1') according to the preceding claim, comprising a gateway device (30) configured to put the monitoring device (10) and the distributed computing environment (20) in mutual data communication.

10. An apparatus (1; 1') according to claim 8 or 9, comprising three radio beacons (50) which are not aligned with one another and each configured to transmit a respective signal, and wherein:
• the sensor group (15) is further configured to detect the respective signal intensities for each of the respective signals emitted by the three radio beacons (50);
• the monitoring device (10) is further configured to transmit the respective signal intensities, and the distributed computing environment (20) is further configured to receive the respective signal intensities;
• the distributed computing environment (20) further comprises a trilateration algorithm for computing the position of the monitoring device (10) as a function of the respective signal intensities;
• the user interface (40) is further configured to provide the user with the position of the monitoring device (10).

11. An apparatus (1; 1') according to any one of claims 8 to 10, wherein the first artificial intelligence model (IA) is further trained to perform at least one of:
• regulating the sampling frequencies of the sensor group (15) increasing and/or decreasing them in response to respective increases and/or decreases in the variation frequencies of the predefined physical parameters;
• estimating current and/or future values of the qualitative and/or quantitative parameters of the wine in the bottle (500) as a function of the respective time series of each of the predefined physical parameters.

12. An apparatus (1; 1') according to any one of claims 8 to 11, wherein the distributed computing environment (20) comprises at least one of:
• a second artificial intelligence model trained to regulate the sampling frequencies of the sensor group (15) increasing and/or decreasing them in response to respective increases and/or decreases in the variation frequencies of the predefined physical parameters;
• a third artificial intelligence model trained to estimate current and/or future values of the qualitative and/or quantitative parameters of the wine in the bottle (500) as a function of the respective time series of each of the predefined physical parameters.

13. Computer-implemented method (100) according to any one of claims 1 to 7, which uses an apparatus (1; 1') according to any one of claims 8 to 12, the method comprising:
N. detecting data relating to an energy consumption of the monitoring device (10) and/or other components of the apparatus (1; 1');
O. analysing the data relating to the energy consumption by means of an artificial intelligence model trained so as to carry out at least one of the sub-steps of:
• defining strategies for optimizing the energy consumption comprising automatically regulating the sampling frequencies of the sensors and/or modulating a communication activity between components of the apparatus (1; 1') in order to extend an operating autonomy of the monitoring device (10);
• recognizing anomalous energy consumption conditions, generating a corresponding warning and consequently providing said warning to the user so as to require a direct intervention of the user aimed at correcting the anomalous energy consumption conditions.

14. Software product for monitoring wine in a bottle (500) during a respective cold chain, comprising instructions which, when carried out by an apparatus (1; 1') according to any one of claims 8 to 12, determine the execution of the steps of a computer-implemented method (100) according to any one of claims 1 to 7 or according to claim 13.

15. A computer-readable medium containing a software product according to the preceding claim.
